(19) Europäisches Patentamt
European Patent Office
Office européen des brevets

(11) **EP 3 150 252 B1**

(12) **FASCICULE DE BREVET EUROPEEN**

(45) Date de publication et mention de la délivrance du brevet:
**19.12.2018 Bulletin 2018/51**

(21) Numéro de dépôt: **16191910.5**

(22) Date de dépôt: **30.09.2016**

(51) Int Cl.:
*A61N 1/37* (2006.01)    *A61B 5/0432* (2006.01)
*A61B 5/07* (2006.01)    *G06F 1/32* (2006.01)
*G06F 19/00* (2018.01)   *A61B 5/00* (2006.01)
*A61B 5/024* (2006.01)

(54) **DISPOSITIF MÉDICAL IMPLANTABLE ACTIF APTE À EFFECTUER UNE ANALYSE FRÉQUENTIELLE**

AKTIVE IMPLANTIERBARE MEDIZINISCHE VORRICHTUNG, DIE IN DER LAGE IST, EINE FREQUENZANALYSE DURCHZUFÜHREN

ACTIVE IMPLANTABLE MEDICAL DEVICE CAPABLE OF PERFORMING FREQUENCY ANALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.10.2015 FR 1559320**

(43) Date de publication de la demande:
**05.04.2017 Bulletin 2017/14**

(73) Titulaires:
• **Sorin CRM SAS**
**92140 Clamart Cedex (FR)**
• **Institut National de la Santé et de la Recherche Médicale (INSERM)**
**75013 Paris (FR)**
• **Université de Rennes 1**
**35065 Rennes Cedex (FR)**

(72) Inventeurs:
• **BONNET, Jean-Luc**
**91300 Massy (FR)**
• **HERRMANN, Thomas**
**75020 Paris (FR)**
• **HENRY, Christine**
**75014 Paris (FR)**
• **HERNANDEZ, Alfredo**
**35510 Cesson Sévigné (FR)**
• **CARRAULT, Guy**
**35510 Cesson Sévigné (FR)**

(74) Mandataire: **Grünecker Patent- und Rechtsanwälte PartG mbB Leopoldstraße 4 80802 München (DE)**

(56) Documents cités:
US-A- 4 404 972    US-A1- 2007 255 331
US-A1- 2007 260 285    US-A1- 2014 088 379
US-A1- 2015 257 669

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle ...

L'invention concerne plus particulièrement un dispositif médical implantable actif apte à recevoir des signaux recueillis par des sondes, ou délivrés par des capteurs et à analyser ces signaux.

La plupart des dispositifs médicaux implantables actifs incluent la capacité de transmettre, notamment à un dispositif externe, des signaux physiologiques comme un électrocardiogramme (ECG) tel que dans US 2015/0257669 A1 et US 2007/0260285 A1, ou un électroneurogramme (ENG), ou un électromyogramme (EMG) tel que dans US 2014/0088379 A1, des signaux relatifs au flux de ventilation ou à l'accélération du rythme cardiaque qui ont été préalablement captés et mémorisés pour analyser ces signaux, comme dans US 2007/0255331 A1.

En particulier, les signaux électrocardiogramme ECG fournissent des données indicatives de l'activité cardiaque, notamment du rythme cardiaque. Le rythme cardiaque est une notion qualitative représentative de la manière dont les cycles se succèdent. Le rythme cardiaque est défini par l'activité électrique cardiaque à l'origine de la contraction du myocarde.

Les signaux électrocardiogramme ECG lorsqu'ils sont affichés en fonction du temps, fournissent une image de l'activité rythmique du coeur. Les problèmes associés à l'activité du coeur sont en général détectés à partir des signaux électrocardiogramme ECG.

L'analyse rythmique du coeur des patients ayant un dispositif implanté est réalisée par exemple à partir des données du signal électrocardiogramme ECG mémorisées dans le dispositif et analysées par ce dispositif. Ces données peuvent être transmises à un dispositif externe utilisé par les cardiologues afin d'être affichées et analysées, en temps réel ou en différé.

Les signaux physiologiques disposent d'informations pertinentes sur l'état physiologique d'un ou plusieurs organes du patient. Notamment, le rythme cardiaque renferme des informations sur le système nerveux autonome, en particulier sur son interrelation avec le système cardiovasculaire. Ces informations peuvent être contenues dans le spectre de fréquences des signaux physiologiques. Une analyse fréquentielle est alors utilisée pour obtenir les informations relatives à l'état physiologique d'un ou plusieurs organes, individuellement ou en interaction.

Il est connu différentes méthodes pour réaliser une analyse fréquentielle (AF) des signaux physiologiques, dont l'une consiste à effectuer une transformée de Fourier Discrète. Une autre méthode d'analyse fréquentielle consiste à effectuer une transformée de Fourier rapide (FFT - « Fast Fourier Transform » en terminologie anglo-saxonne), de manière à exploiter certaines optimisations de l'opération de la transformation de Fourier Discrète.

Il est également connu de procéder à des analyses en échelle dite analyse en ondelettes, par exemple par l'analyse en ondelettes discrètes. Ces méthodes disposent aussi de version de calcul rapide.

Les analyses fréquentielles sont principalement mises en oeuvre dans des systèmes ayant un microprocesseur ou un microcontrôleur non limité par la consommation d'énergie, notamment un ordinateur, pour lesquels un traitement rapide et continu est fondamental.

Par exemple, il est connu du document WO 91/19452 l'utilisation de la transformation de Fourier rapide (FFT) pour l'analyse des signaux issus de l'électrocardiogramme par un ordinateur externe après acquisition des signaux sur le patient pour le diagnostic des arythmies cardiaques.

En outre, différentes solutions ont été proposées pour l'analyse de signaux captés par une transformation de Fourier rapide. En effet, il est connu d'une part d'utiliser un circuit dédié aussi appelé processeur de signal numérique (ou « Digital Signal Processor » en terminologie anglo-saxonne) et d'autre part d'utiliser un microprocesseur standard capable de traiter des calculs de haut niveau. Toutefois, ces deux solutions présentent des inconvénients.

En effet, il est connu, notamment du document US 7,186,220, une solution consistant à utiliser un circuit dédié, c'est-à-dire un microprocesseur optimisé pour exécuter des applications de traitement numérique du signal. Cette solution permet un calcul rapide mais implique lors de son implantation dans un dispositif médical implantable autonome plusieurs difficultés à résoudre. Tout d'abord, une telle implantation nécessite un emplacement physique important alors que la taille du dispositif médical implantable est cruciale et doit être la plus petite possible. En outre, la consommation d'énergie importante des circuits dédiés présente un impact sur la durée de vie de ces dispositifs médicaux implantables. Les dispositifs médicaux implantables actifs devant être aussi petits que possible pour une implantation dans le corps humain ne peuvent accueillir un tel circuit. Ainsi, la solution consistant à implémenter un circuit dédié dans un tel dispositif médical implantable nécessite trop d'espace et est consommatrice d'énergie dans la mesure où le traitement est effectué en continu.

Une autre solution consistant à utiliser un microprocesseur ou microcontrôleur standard, c'est-à-dire utilisé pour réaliser les fonctions habituelles du système implantable, évite de réaliser des modifications matérielles pour la mise en oeuvre de l'analyse fréquentielle. Toutefois, ces microprocesseurs ou microcontrôleurs ont des performances limitées en termes de puissance de calcul. A titre d'exemple, la complexité de calcul d'une transformée de Fourier rapide varie suivant O(n.log n). De tels calculs nécessitent un très grand nombre de cycles de calcul d'un microprocesseur ou d'un microcontrôleur.

Ainsi, le calcul d'une analyse fréquentielle nécessite une quantité d'énergie importante et une unité de calcul puissante, ces critères prérequis ne sont pas remplis par les dispositifs médicaux implantables actifs.

L'un des buts de l'invention est de proposer une solution à ces difficultés avec un dispositif médical implantable actif pour la réalisation d'une analyse fréquentielle des signaux, notamment par une transformation de Fourier, au sein du dispositif médical sans modifier son architecture aussi bien matériel que logiciel.

A cet effet, l'invention propose un dispositif médical implantable actif comprenant une unité de traitement apte à être mise alternativement en fonctionnement durant une période d'activité déterminée et en veille durant une période de veille de manière cyclique, et des moyens d'acquisition de données liées à l'activité physiologique et/ou physique.

De façon caractéristique, le dispositif comprend en outre des moyens de calcul d'une analyse fréquentielle des données acquises, lesdits moyens de calcul étant aptes à exécuter successivement une partie de l'analyse fréquentielle durant des périodes d'activité de l'unité de traitement, et des moyens d'optimisation de l'analyse fréquentielle par fractionnement de l'analyse fréquentielle en une pluralité de modules élémentaires, les moyens de calcul exécutant successivement les modules élémentaires durant des périodes d'activité de l'unité de traitement.

Selon diverses caractéristiques subsidiaires :

- les moyens de calcul sont mis en oeuvre au cours de la période d'activité de l'unité de traitement après exécution des fonctions standards de traitement exécutées durant la période d'activité de l'unité de traitement.
- les moyens de calcul exécutent une partie de l'analyse fréquentielle après exécution des fonctions standards de traitement exécutées jusqu'à la fin de la période d'activité de l'unité de traitement.
- lesdits moyens de calcul exécutent une partie de l'analyse fréquentielle durant une durée prédéterminée.

- les moyens d'optimisation de l'analyse fréquentielle sont aptes en outre à sélectionner au moins une bande de fréquence à traiter.
- le dispositif comprend en outre des moyens de sélection d'une période d'acquisition desdites données.
- les moyens de sélection d'une période d'acquisition comprennent en outre des moyens de vérification de conformité de critères prédéterminés.
- les moyens d'acquisition des données sont mis en oeuvre lorsque la période d'acquisition est atteinte et que les critères sont conformes à des valeurs prédéterminées.
- le dispositif comprend en outre des moyens de détection d'un événement physiologique déterminé externe au dispositif et des moyens de démarrage du cycle apte à démarrer le cycle lorsque les moyens de détection détectent un événement physiologique déterminé.
- le dispositif comprend en outre des moyens de prétraitement des données acquises.
- les moyens de prétraitement sont aptes à interpoler et rééchantillonner les données acquises.
- les moyens de calcul sont mis en oeuvre dans une partie finale de la période d'activité de l'unité de traitement.
- l'analyse fréquentielle est une transformation de Fourier.
- les moyens de prétraitement sont aptes à sélectionner un sous-ensemble des données acquises.
- les moyens de prétraitement sont aptes à filtrer les données acquises.
- le signal acquis est l'électrocardiogramme (ECG).
- le signal acquis est l'électrogramme endocavitaire (EGM).
- le signal acquis est l'intervalle temporel entre deux évènements cardiaques consécutifs.
- le signal d'un accéléromètre endocavitaire cardiaque (EA).
- le signal acquis est l'électroneurogramme (ENG).
- le signal acquis est l'électromyogramme (EMG).
- le signal acquis est la ventilation.
- le signal acquis est la contraction gastrique.
- le signal acquis est un signal d'activité physique tel que l'accélération.
- l'analyse fréquentielle est effectuée par une transformation de Fourier rapide (FFT).
- l'analyse fréquentielle est une analyse de puissance spectrale.
- l'analyse fréquentielle est substituée par une analyse d'ondelettes.

On va maintenant décrire un exemple de mise en oeuvre du dispositif de l'invention, en référence aux dessins annexés.

La figure 1 est une vue générale de présentation d'un dispositif médical implantable actif.

La figure 2 est une méthode pour l'analyse fréquentielle de données conformément à l'invention.

La figure 3 illustre le séquentiellement de l'analyse fréquentielle selon l'invention.

La figure 4 est un exemple d'optimisation de l'analyse fréquentielle selon l'invention.

La figure 5 est un premier mode de réalisation des moyens de calcul d'une analyse fréquentielle conformément à l'invention.

La figure 6 est un deuxième mode de réalisation des moyens de calcul d'une analyse fréquentielle conformément à l'invention.

La figure 7 est un troisième mode de réalisation des moyens de calcul d'une analyse fréquentielle conformément à l'invention.

La figure 8 illustre un exemple de mise en oeuvre d'une analyse des intervalles R-R du rythme cardiaque d'un patient.

La figure 9 illustre des fréquences sur lesquelles l'analyse fréquentielle conformément à l'invention peut être réalisée.

La figure 10 illustre des rapports de fréquences sur lesquels l'analyse fréquentielle conformément à l'invention peut être réalisée.

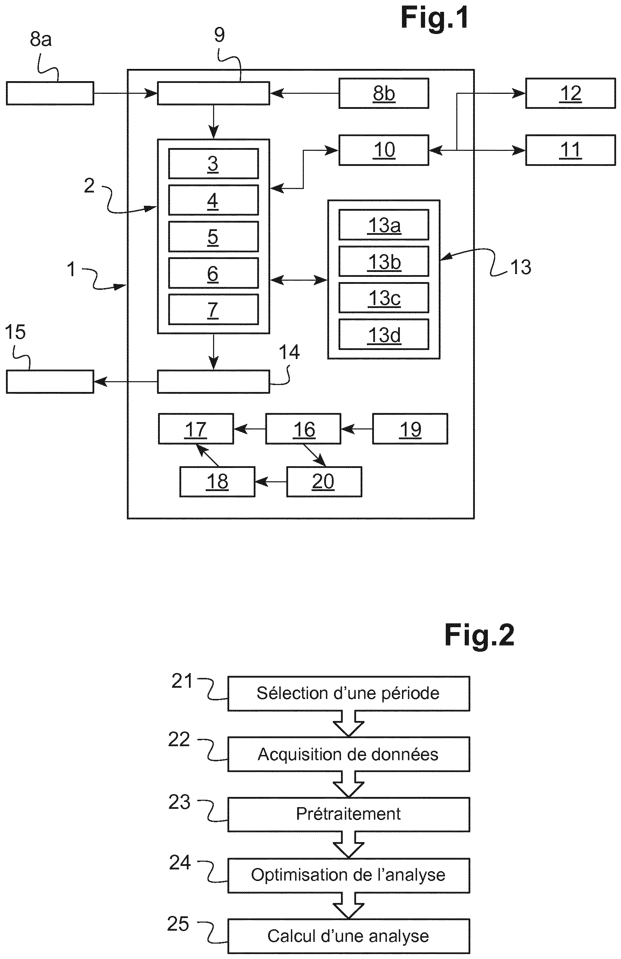

Un dispositif médical implantable actif 1 est illustré en figure 1. Ce dispositif comporte une unité de traitement 2, notamment, un processeur, un micro-processeur ou un microcontrôleur comportant une mémoire interne de travail 3.

L'unité de traitement comprend une horloge 4 propre dont la fréquence détermine la vitesse de fonctionnement de l'unité de traitement. Cette dernière comprend en outre par exemple des algorithmes standards 5, notamment la gestion des épisodes bradycardie et tachycardie, un algorithme d'analyse fréquentielle 6, un algorithme d'asservissement 7, etc.

Au moins un capteur 8a ou 8b de paramètres physiologiques et physiques, tels que les dérivations cardiaques, le rythme cardiaque, etc., tel que l'accéléromètre permettant de connaître l'activité physique, la position et l'activité cardiaque du patient, est connecté par exemple à une unité de traitement des capteurs 9 du dispositif médical implantable actif 1 afin de transmettre au dispositif médical des données physiologiques ou physiques captées sur le patient.

D'autres capteurs peuvent également être implantés au sein du patient, par exemple pour recueillir un électrogramme endocavitaire (EGM), un électroneurogramme (ENG), un électromyogramme (EMG), un signal de contractilité cardiaque comme l'accélération endocavitaire (EA), un signal de contractilité gastrique, ou tout autre signal physiologique ou physique tel que le signal d'accélération corporelle.

En outre, le dispositif médical implantable actif 1 peut comprendre un système de télémétrie intégrant un appareil de communication interne 10, par exemple du type communication inductive ou radio fréquence (RF) permettant une communication avec un dispositif externe 11, notamment un ordinateur ou tout autre dispositif tel qu'un système de surveillance 12.

Le dispositif médical implantable actif 1 comprend de plus un dispositif de mémoire de stockage 13 comprenant un espace de stockage pour la mémorisation de données variées, par exemple des données brutes 13a, des données échantillonnées 13b, des données temporaires 13c ou des données ayant subies un traitement 13d.

Un tel dispositif médical implantable actif 1 peut aussi comprendre un système d'interaction 14 avec un appareil externe 15, le système d'interaction étant apte à délivrer la thérapie adaptée demandée par l'appareil externe 15, cette thérapie pouvant être par exemple la stimulation cardiaque ou la délivrance de médicament à travers une pompe.

De manière récurrente, l'unité de traitement du dispositif médical implantable actif est apte à être mise alternativement en fonctionnement durant une période d'activité déterminée et en veille durant une période de veille.

Selon l'invention, la période d'activité est d'une durée fixée et prédéterminée ou variable et la période de veille est d'une durée fixée ou variable en fonction de l'activité physiologique.

La durée de la période d'activité additionnée à la durée de la période de veille correspond à la durée totale d'un cycle.

Selon l'invention, le cycle peut être régulier ou irrégulier.

Par exemple, dans une application cardiaque, le cycle est fonction de l'activité cardiaque du patient. En revanche, dans le cas d'un électromyogramme (EMG), la durée des périodes d'activité et de veille peut être fixée au préalable.

Selon un mode de réalisation particulier, le cycle correspond au cycle cardiaque du patient. Ainsi, l'unité de traitement est dans un état de veille et est réveillée à chaque cycle cardiaque, par exemple à chaque début de cycle cardiaque. Selon ce mode de réalisation, il y a synchronisation de l'activation de l'unité de traitement du dispositif médical implantable actif avec un événement cardiaque.

Pour ce faire, le dispositif médical comprend des moyens de détection d'un événement physiologique déterminé externe au dispositif et des moyens de démarrage du cycle apte à démarrer le cycle lorsque les moyens de détection détectent un événement physiologique déterminé, par exemple le début du cycle cardiaque.

L'unité de traitement est alors en fonctionnement durant une période d'activité déterminée.

Selon un exemple de réalisation, la période d'activité est la période réfractaire, c'est-à-dire la période qui succède immédiatement à l'activité d'un événement cardiaque.

Selon un exemple illustratif, lorsque la fréquence cardiaque est de 60 battements par minute, alors le cycle cardiaque est de 1000 millisecondes. L'unité de traitement est réveillée à chaque début de cycle cardiaque pour une durée déterminée, par exemple de 16 millisecondes.

Selon un autre exemple illustratif, la durée de traitement est variable en fonction de la charge de calcul nécessaire au cycle, dans la limite d'une valeur maximale, notamment afin de ne pas dépasser la période réfractaire cardiaque.

Un exemple de mode de fonctionnement cyclique de l'unité de traitement est détaillé dans le document FR2492262.

Ainsi, selon l'invention, l'unité de traitement est sollicitée uniquement pendant quelques millisecondes par cycle ce qui a pour avantage une consommation d'énergie très faible par cycle, puisque l'unité de traitement est en état de veille pendant la majorité du temps. La consommation d'énergie et donc la sollicitation de la pile du dispositif médical implantable est donc répartie sur une fraction de chaque cycle.

Durant la période d'activité de l'unité de traitement, celle-ci exécute des fonctions standards de traitement. L'exécution de ces fonctions standards de traitement est réalisée durant une durée non constante, la durée d'exécution étant dépendante des données captées sur le patient et du temps d'exécution des fonctions standards.

Selon l'invention, le dispositif médical implantable actif comprend des moyens d'acquisition de données 16 liées à l'activité physiologique.

L'activité physiologique est par exemple le rythme cardiaque du patient. Toutefois, l'activité physiologique peut être un signal lié à la ventilation, à un électroneurogramme, à un électromyogramme, à un électrocardiogramme, à l'accélération ou tout autre signal physiologique ou physique lié au patient.

Pour ce faire, les moyens d'acquisition peuvent obtenir les données relatives à l'activité physiologique du patient via par exemple les capteurs 8a ou 8b reliés au dispositif médical implantable actif 1. L'acquisition des données est réalisée par exemple à un moment particulier de la journée ou de la semaine.

En outre, selon l'invention, le dispositif médical implantable actif comprend des moyens de calcul d'une analyse fréquentielle 17. Ces moyens de calcul sont par exemple le calcul d'une transformée de Fourier, d'une analyse en Ondelettes ou d'une analyse de type temps-fréquence. Les moyens de calcul 17 réalisent les calculs à partir des données acquises afin d'effectuer une analyse de ces données.

Afin d'être apte à exécuter une analyse fréquentielle dans un tel dispositif médical implantable actif tout en maintenant la durée de vie du dispositif et ces fonctions principales, celui-ci comprend des moyens de calcul 17 aptes à exécuter successivement une partie de l'analyse fréquentielle durant des périodes d'activité de l'unité de traitement 2.

Ainsi, conformément à l'invention, l'analyse fréquentielle des données est fractionnée pour être exécutée par partie ou fragment durant des périodes d'activité de l'unité de traitement.

Une analyse fréquentielle optimisée va maintenant être décrite conformément à l'invention.

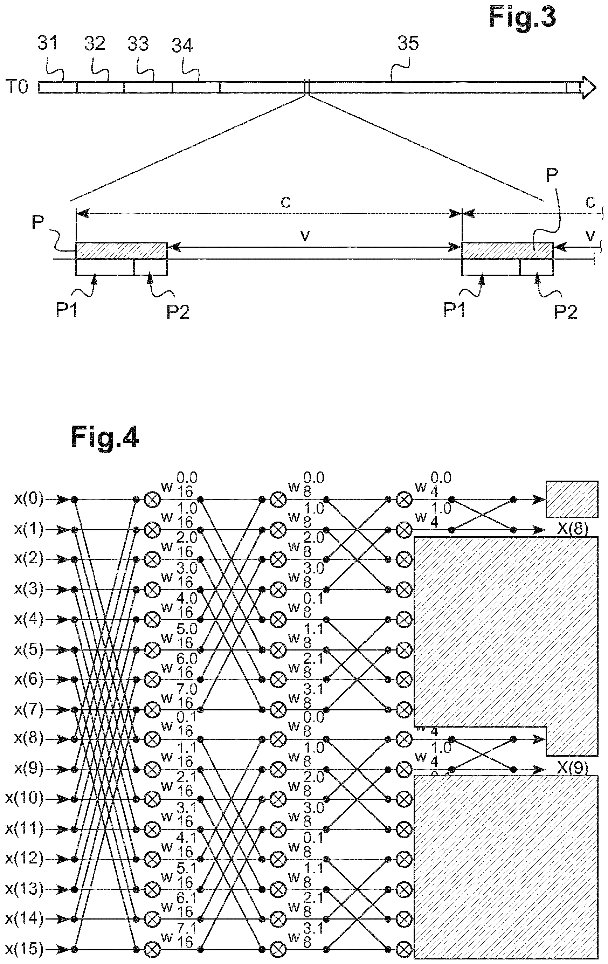

L'exécution fragmentée de l'analyse fréquentielle par le dispositif médical implantable actif est détaillée ci-dessous et illustrée en figure 2. Le séquencement temporel de l'analyse fréquentielle de données est également illustré en figure 3.

Cette analyse débute par une étape 21 qui est une étape de sélection d'une période d'acquisition des données liées à l'activité physiologique, et/ou physique du patient et/ou à des données externes. Par exemple, la sélection d'une période d'acquisition est liée à l'activité cardiaque et / ou l'activité physique du patient.

Une période d'acquisition des données est, par exemple en fonction d'une information temporelle prédéterminée.

Lors de cette étape, la conformité de critères prédéterminés peut également être vérifiée, ces critères étant notamment des conditions physiologiques.

Un exemple de critère est l'absence d'activité physique du patient durant l'acquisition des données, l'activité physique étant notamment mesurée à partir du capteur d'accéléromètre (G) et du rythme cardiaque.

Les critères peuvent aussi correspondre à des périodes d'activité physique du patient, par exemple dans le cas de l'électromyogramme.

D'autres exemples de critères prédéterminés sont l'absence de stimulation par le dispositif et l'absence d'arythmie cardiaque.

Le critère peut aussi être uniquement basé sur l'heure de la journée, par exemple pour enregistrer un signal à une heure donnée et fixée à l'avance.

Cette étape est illustrée en figure 3 par la référence 31.

Lorsque la période d'acquisition des données est atteinte et que les critères prédéterminés sont remplis alors l'étape 21 est suivie d'une étape d'acquisition 22 des données. L'acquisition des données est réalisée notamment par les moyens d'acquisition de données 16 précédemment décrits.

Durant cette étape, les données acquises correspondent par exemple, à des durées d'intervalles de temps entre deux débuts de cycle.

Selon un exemple particulier d'analyse d'intervalles R-R, c'est-à-dire du temps entre deux ondes R consécutives du rythme cardiaque, l'étape d'acquisition des données consiste par exemple en l'acquisition de 1024 intervalles de temps entre deux ondes R consécutives.

Ces données acquises sont par exemple mémorisées dans le dispositif mémoire de stockage 13.

Cette étape est illustrée en figure 3 par la référence 32.

Selon un mode de réalisation particulier, l'étape d'acquisition des données peut être interrompue suite à la survenue d'un ou plusieurs critères liés au patient (rythme cardiaque, activité physique, température...) ou à un événement externe (heure, commande par télémétrie...).

L'étape 22 est suivie d'une étape 23 de prétraitement des données acquises.

Le prétraitement des données consiste à appliquer des techniques de traitement du signal pour sa mise en forme avant l'analyse fréquentielle. Le prétraitement peut consister par exemple en l'une ou une combinaison des actions suivantes : filtrer, redresser, moduler, démoduler, sur-échantillonner, sous-échantillonner, interpoler...

Selon un exemple particulier, lors de cette étape, les durées des intervalles de temps entre deux ondes R acquises sont converties en une donnée de temps constante par interpolation et par ré-échantillonnage et une sélection d'un ensemble d'échantillons est réalisée.

Le ré-échantillonnage est réalisé par exemple à une fréquence de 4Hz.

Un autre exemple de prétraitement des données acquises consiste à corriger les durées des intervalles de temps R-R lors de contractions ventriculaires prématurées aussi appelées extrasystoles ventriculaires.

Durant cette étape de prétraitement, il peut être aussi sélectionné un sous ensemble des données acquises parmi l'ensemble des données acquises.

Cette étape est illustrée en figure 3 par la référence 33.

L'étape 33 est suivie de l'étape 34 d'optimisation de l'analyse fréquentielle.

Pour ce faire, des bandes de fréquence sur lesquelles vont être appliquées l'analyse fréquentielle, notamment la transformation de Fourier, sont sélectionnées.

En figure 4, il est illustré un exemple d'optimisation de l'analyse fréquentielle par la suppression de bandes de fréquence, les bandes de fréquence supprimées étant représentées en gris. En effet, il a été observé que le signal pertinent est présent dans seulement quelques bandes de fréquence. Un exemple de bande de fréquence est la fréquence sinusale comprise entre 0,5 et 3 Hz.

En sélectionnant des bandes de fréquence, le calcul de l'analyse fréquentielle est limité au(x) bande(s) de fréquence cible(s) réduisant de la sorte la durée de l'analyse fréquentielle.

En outre, lors de cette étape et selon un mode de réalisation particulier, l'analyse fréquentielle est fractionnée en une pluralité de parties appelées dans ce mode de réalisation particulier, modules élémentaires me.

Par exemple, l'analyse d'un électrocardiogramme nécessite l'exécution de 50.000 modules élémentaires me durant 50.000 cycles cardiaques.

Selon un autre exemple, l'analyse d'un électromyogramme nécessite l'exécution de 80.000 modules élémentaires me soit 80.000 cycles, notamment un cycle par seconde, soit 80.000 secondes (un peu moins de 24 heures).

Cette étape est illustrée en figure 3 par la référence 34.

L'étape 24 est suivie d'une étape 25 de calcul d'une analyse fréquentielle sur lesdites données par exécution successive des parties ou modules élémentaires de l'analyse fréquentielle durant des périodes d'activité de l'unité de traitement.

Ainsi l'analyse des données est répartie sur une pluralité de cycles.

Les moyens de calcul de l'analyse fréquentielle 17 sont aptes à exécuter les parties ou modules élémentaires me de l'analyse fréquentielle.

Cette étape est illustrée en figure 3 par la référence 35.

Pour mettre en oeuvre l'analyse fréquentielle, le dispositif médical implantable actif peut comprendre des moyens de sélection 19 d'une période d'acquisition desdites données. Les moyens de sélection d'une période d'acquisition peuvent comprendre en outre des moyens de vérification de conformité de critères prédéterminés tels que décrits précédemment.

En outre, les moyens d'acquisition des données 16 précédemment décrits peuvent être mis en oeuvre lorsque la période d'acquisition est atteinte et que les critères sont conformes à des valeurs prédéterminées

De plus, le dispositif médical implantable actif peut comprendre des moyens de prétraitement 20 des données acquises aptes à rééchantillonner les durées d'intervalles de temps entre deux débuts de cycle acquises selon une donnée temporelle constante et à sélectionner une séquence d'échantillons.

Ces moyens de prétraitement 20 peuvent aussi réaliser une sélection d'un sous-ensemble de données acquises.

Enfin, le dispositif médical implantable actif peut comprendre des moyens d'optimisation 18 de l'analyse fréquentielle aptes à sélectionner au moins une bande de fréquence à traiter tel que précédemment décrit.

La figure 3 montre également un séquencement de l'état de fonctionnement de l'unité de traitement du dispositif médical implantable actif durant deux cycles c et l'exécution de parties de l'analyse fréquentielle.

Cette figure illustre deux activations successives de l'unité de traitement, l'activation étant réalisée par exemple de manière synchrone avec un événement cardiaque.

Lors d'une activation de l'unité de traitement, celle-ci va être maintenue active durant une période d'activité p d'une durée prédéterminée, par exemple 16 millisecondes.

Durant une première partie p1 de cette période d'activité, l'unité de traitement exécute des fonctions standards de traitement.

Durant une seconde partie p2 de cette période d'activité, l'unité de traitement exécute une partie de l'analyse fréquentielle.

A la fin de la période d'activité de l'unité de traitement, celle-ci passe dans un état de veille durant une période de veille v correspondant à la période d'inactivité, cette période se prolongeant jusqu'à la fin du cycle c.

Ainsi, les moyens de calcul 17 d'une analyse fréquentielle d'un dispositif médical actif conformément à l'invention exécutent des parties de l'analyse fréquentielle au cours de la période d'activité p2 de l'unité de traitement.

En particulier, l'exécution des parties de l'analyse fréquentielle est réalisée après l'exécution des fonctions standards de traitement, c'est-à-dire au cours de la seconde partie p2 de la période d'activité de l'unité de traitement. En d'autres termes, les parties de l'analyse fréquentielle sont exécutées à la fin de la période d'activité p de l'unité de traitement.

Selon une alternative de réalisation, les moyens de calcul du dispositif médical implantable actif exécutent les parties de l'analyse fréquentielle au début de la période d'activité de l'unité de traitement.

Ainsi, l'invention permet avantageusement d'utiliser les périodes d'activité de l'unité de traitement pour réaliser l'analyse fréquentielle. En outre, l'invention est basée sur l'architecture existante des dispositifs médicaux implantables, et ne compromet pas l'exécution des fonctions principales du dispositif notamment en respectant le temps réel nécessaire à l'exécution des fonctions du dispositif. L'invention permet donc une optimisation des ressources nécessaires à l'analyse, sans augmenter significativement la consommation d'énergie, c'est-à-dire sans diminuer la durée de vie du dispositif médical, sans compromettre le traitement temps-réel des fonctions usuelles, et enfin sans modifier l'aspect matériel, notamment sans l'ajout d'un circuit de traitement dédié.

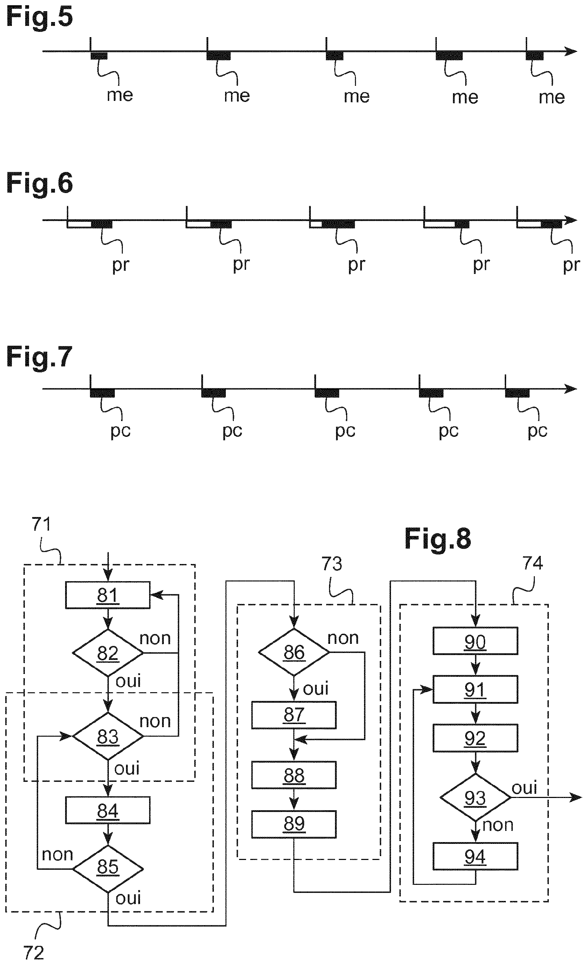

Selon un premier mode de réalisation des moyens de calcul d'une analyse fréquentielle conformément à l'invention illustré en figure 5, le dispositif médical implantable actif comprend des moyens d'optimisation de l'analyse fréquentielle 18 par fractionnement de l'analyse fréquentielle en une pluralité de modules élémentaires *me*. Les parties exécutées par les moyens de calcul sont dans ce mode de réalisation, les modules élémentaires *me* issus du fractionnement de l'analyse fréquentielle.

Ces modules élémentaires me peuvent être de tailles différentes tel qu'illustré en figure 5. Ainsi, la durée d'exécution de chaque module élémentaire *me* est différente. Toutefois, le fractionnement de l'analyse fréquentielle est réalisé de telle sorte que la durée d'exécution de chaque module élémentaire soit inférieure à une durée déterminée, par exemple 2 millisecondes. Ainsi, la durée maximale d'exécution de chaque module élémentaire me est connue.

Bien entendu, la fragmentation de l'analyse fréquentielle est réalisée en fonction de la durée estimée de la période d'activité restante après le traitement des fonctions standards.

Conformément à l'invention, chaque module élémentaire me est exécuté successivement durant les périodes d'activité de l'unité de traitement, notamment à chaque période d'activité de l'unité de fonctionnement sans interruption.

Alternativement, les modules élémentaires me sont exécutés durant des périodes d'activités de l'unité de fonctionnement non consécutives. Ce mode de réalisation est avantageux lorsque l'exécution des fonctions standards de traitement dans un cycle donné est réalisée dans un temps ne permettant plus l'exécution complète d'un module élémentaire me durant la période d'activation de l'unité de traitement. Pour ce faire, la durée de la seconde partie de la période d'activité de l'unité de traitement est calculée, si celle-ci est inférieure à la durée maximale d'exécution d'un module élémentaire *me*, alors les moyens de calcul de l'analyse fréquentielle 17 attendront la prochaine période d'activité de l'unité de traitement pour exécuter la partie de l'analyse fréquentielle.

Selon un deuxième mode de réalisation des moyens de calcul d'une analyse fréquentielle conformément à l'invention illustré en figure 6, les moyens de calcul exécutent une partie *pr* de l'analyse fréquentielle après l'exécution des fonctions standards, l'exécution de l'analyse fréquentielle se poursuit jusqu'à la fin de la période d'activité de l'unité de traitement.

Ainsi, l'analyse fréquentielle débute après l'exécution des fonctions standards de traitement et se prolonge durant toute la durée restante de la période d'activité p de l'unité de traitement tel qu'illustré en figure 6.

Selon ce mode de réalisation, la durée d'exécution de chaque partie de l'analyse fréquentielle durant les périodes d'activation de l'unité de traitement est différente puisque cette partie *pr* est dépendante du temps restant de la période d'activité p2 de l'unité de traitement après exécution des fonctions standards.

Ce mode de réalisation présente l'avantage d'utiliser l'intégralité de la période d'activité de l'unité de traitement et donc d'optimiser le temps d'exécution de l'analyse fréquentielle.

Selon un troisième mode de réalisation des moyens de calcul d'une analyse fréquentielle conformément à l'invention, illustré en figure 7, les moyens de calcul exécutent successivement à chaque période d'activité de l'unité de traitement une partie *pc* de l'analyse fréquentielle durant une durée prédéterminée.

Selon ce mode de réalisation, la durée d'exécution de chaque partie *pc* de l'analyse fréquentielle est déterminée, par exemple, cette durée est de 2 millisecondes. A la fin de cette durée, l'exécution de l'analyse fréquentielle est arrêtée.

Les moyens de calcul peuvent exécuter la partie *pc* de l'analyse fréquentielle de manière préférentielle après l'exécution des fonctions standards. Toutefois, de manière alternative, la partie *pc* de l'analyse fréquentielle peut être exécutée au début de la période d'activité p de l'unité de traitement.

La figure 8 illustre un exemple de mise en oeuvre selon l'invention, d'une analyse des intervalles R-R d'un patient selon une analyse fréquentielle particulière, à savoir une transformation de Fourier.

Cette mise en oeuvre est basée sur une étape 71 de sélection de la période d'acquisition conformément à l'étape 21 de la figure 2, puis d'une étape 72 d'acquisition de données conformément à l'étape 22 de la figure 2 puis d'une étape 73 de prétraitement conformément à l'étape 23 de la figure 2 et enfin d'une étape 74 de calcul d'une analyse fréquentielle sur des données acquises conformément à l'étape 25 de la figure 2.

Il va être maintenant décrit en détail chacune de ces étapes lors de leur mise en oeuvre pour l'analyse d'intervalles R-R en utilisant une transformation de Fourier.

Les étapes décrites ci-dessous sont exécutées de manière séquentielle.

L'étape 71 de sélection de la période d'acquisition se décompose en sous-étapes 81 à 83.

La première étape 81 est une étape d'attente de la période de temps prédéterminée à laquelle l'acquisition des données sur le patient doit avoir lieu.

L'étape 81 est suivie d'une étape 82 de test afin de déterminer si la période de temps d'acquisition est atteinte.

Si la période de temps d'acquisition n'est pas atteinte, alors l'algorithme se poursuit à l'étape 81 précédemment décrite.

Dans le cas contraire, l'étape 82 se poursuit à l'étape 83 permettant de tester des critères prédéterminés, notamment les conditions physiologiques du patient par rapport à des valeurs prédéterminées.

Si l'ensemble des critères prédéterminés captés sur le patient ne sont pas conformes aux valeurs prédéterminées, alors l'algorithme se poursuit à l'étape 81 précédemment décrite, notamment afin d'attendre la prochaine période de temps d'acquisition.

Dans le cas contraire, l'étape 83 se poursuit à l'étape 84 d'acquisition de données, ces données étant les durées des intervalles R-R durant un temps donné et d'enregistrement de ces durées.

L'étape 84 se poursuit à l'étape 85 permettant de déterminer si l'enregistrement des données est complet.

Les étapes 84 et 85 sont des sous étapes de l'étape 72 d'acquisition de données.

Si l'enregistrement est incomplet, alors l'algorithme se poursuit à l'étape 83 précédemment décrite.

En effet, l'acquisition et l'enregistrement des données sont arrêtés dès lors qu'un critère prédéterminé capté sur le patient n'est pas conforme à la valeur déterminée. Si tel est le cas, une nouvelle période de temps d'acquisition sera attendue pour réaliser l'acquisition des données, cette nouvelle période pouvant être le jour suivant.

Si au contraire, l'activité physiologique du patient est telle que les critères sont remplis durant toute la période d'acquisition alors l'acquisition se termine lorsque la période d'acquisition prédéfinie est terminée ou que la capacité de stockage est atteinte.

Dans ce cas, l'étape 85 est suivie d'une étape 86 qui est une première sous-étape de l'étape 73 de prétraitement des données acquises.

L'étape 86 est une étape de test permettant de déterminer si des contractions ventriculaires prématurées sont détectées.

Si de telles contractions sont détectées, alors l'étape 86 est suivie d'une étape 87 de correction des durées des intervalles R-R durant lesquelles des contractions ventriculaires prématurées ont été détectées.

Cette étape 87 est suivie d'une étape 88 de ré-échantillonnage des durées des intervalles R-R en une information temporelle compatible avec le calcul de la transformation de Fourier, par exemple par échantillonnage à une fréquence de 4 Hz.

Si lors de l'étape 86, aucune contraction ventriculaire prématurée n'est détecté, alors l'étape 86 se poursuit à l'étape 88 de ré-échantillonnage des durées des intervalles R-R.

L'étape 88 se poursuit à l'étape 89 de sélection d'un ensemble d'échantillons, notamment il est sélectionné $2^n$ échantillons qui vont être traités par la transformation de Fourier, par exemple 1024 échantillons.

Les étapes suivantes constituent des sous-étapes de l'étape 74 de calcul d'une analyse fréquentielle sur des échantillons par exécution successive de parties de la transformation de Fourier, notamment de modules élémentaires.

Pour ce faire, l'étape 89 se poursuit à l'étape 90 d'initialisation de la transformation de Fourier et du compteur i à 0.

L'étape 90 se poursuit à l'étape 91 d'attente de la fin d'exécution des fonctions standards de traitement par l'unité de traitement durant la période d'activité.

Lorsque la fin d'exécution des fonctions standards de traitement est détectée, l'étape 91 se poursuit à l'étape 92 de calcul d'une partie de la transformation de Fourier, notamment d'un module élémentaire.

Cette étape 92 se poursuit à l'étape 93 de test du compteur i afin de déterminer si l'ensemble de la transformation de Fourier a été traité, notamment si l'ensemble des modules élémentaires ont été exécutés.

Le nombre de modules élémentaires est défini dans cet exemple par la variable m.

Si le compteur i est inférieur au nombre de modules élémentaires m, alors l'algorithme se poursuit à l'étape 94 par une incrémentation du compteur i de la valeur 1. Puis l'algorithme se poursuit à l'étape 91 précédemment décrite afin d'exécuter la partie de la transformation suivante, notamment le module élémentaire suivant.

Lorsque tous les modules élémentaires de la transformation de Fourier sont exécutés, le compteur i est égal à la variable m lors de l'étape 93, alors l'algorithme se termine et le résultat de la transformation de Fourier peut être exploité et l'algorithme de la figure 8 peut être de nouveau exécuté.

Le dispositif médical implantable actif conformément à l'invention peut être mis en oeuvre pour l'analyse fréquentielle de différents types de signaux ou données, notamment :

-   pour l'analyse de la variabilité du rythme cardiaque par extraction des composantes basses fréquences et hautes fréquences dans le rythme cardiaque,
-   pour l'analyse de la fréquence de l'électroneurogramme à partir d'une caractérisation des types de fibres,
-   pour l'analyse des oscillations de pression artérielle, par exemple de l'oscillation de Traube-Hering-Mayer,
-   pour l'analyse de troubles respiratoires, par exemple la respiration de Cheyne-Stokes,
-   pour l'analyse de contractions gastriques,
-   pour l'analyse de données d'un électromyogramme, par exemple pour la discrimination entre le signal cible et l'activité musculaire, et
-   pour l'analyse de champs distants, par exemple pour la discrimination entre le signal cible et le bruit distant.

Dans une application particulière de l'invention, l'analyse fréquentielle est réalisée sur la variabilité du rythme cardiaque. Celle-ci permet, entre autres, de détecter des dysfonctionnements physiologiques. L'une de ces dysfonctions concerne une altération de la balance sympatho-vagale, représentant l'équilibre entre le système autonomique parasympathique (nerf vague) et le système sympathique. Ce déséquilibre est reconnu comme étant un facteur prédictif de mortalité dans certaines pathologies cardiovasculaires, en particulier post-infarctus du myocarde.

La variabilité du rythme cardiaque peut être appréciée au travers de divers paramètres. Ces paramètres peuvent concerner le domaine temporel basé sur les mesures de puissance dans des bandes prédéfinies telles que les bandes hautes fréquences (HF) et basses fréquences (LF), etc.

Le ratio hautes fréquences sur basses fréquences (LF/HF) est considéré comment étant la valeur la plus représentative de la balance sympatho-vagale (SVB).

Dans le domaine clinique, la technique de Holter permet l'enregistrement de l'activité cardiaque d'un sujet pendant une durée de temps, par exemple 24 ou 48 heures et permet une analyse du rythme cardiaque du sujet.

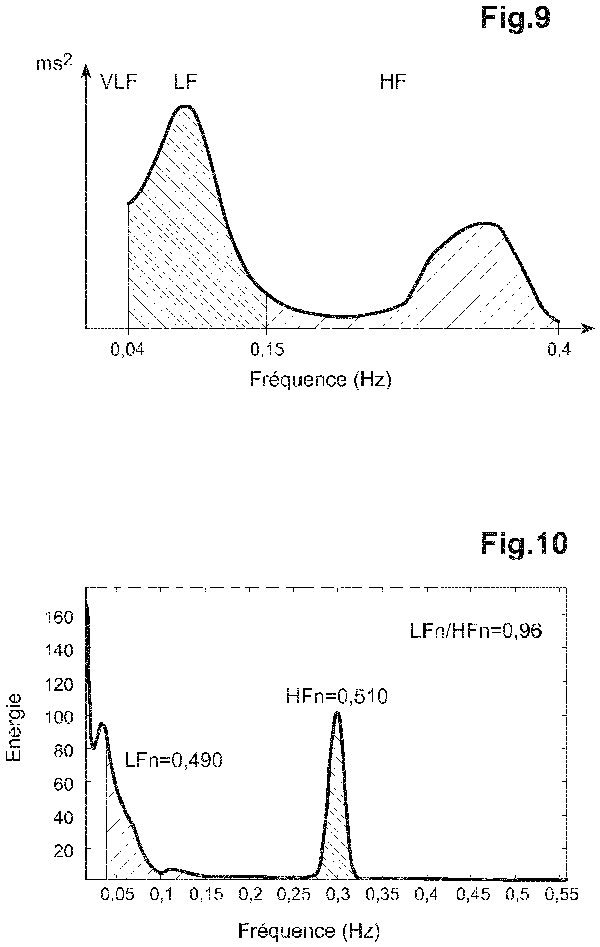

Les figures 9 et 10 illustrent des fréquences sur lesquelles peuvent être appliqués l'analyse fréquentielle appliquée à l'activité rythmique du coeur conformément à l'invention.

L'analyse des domaines de fréquence est normalisée tel que montré en figure 9. Ainsi les hautes fréquences (HF) chez l'adulte concernent la bande de fréquence de 0,15 à 0,4 Hz et les basses fréquences (LF) concernent la bande de fréquence de 0,04 à 0,15 Hz. Les hautes fréquences sont un indicateur de l'activité parasympathique et les basses fréquences traduisent principalement l'activité sympathique. Le ratio LF/HF est un indicateur global de la balance sympatho-vagale SVB.

La balance sympatho-vagale est souvent estimée à partir de la puissance dans ces deux bandes de fréquences et les valeurs peuvent être normalisées selon les formules suivantes :

$$LF = \int_{0,04}^{0,15} S(f)\,df$$

$$HF = \int_{0,15}^{0,4} S(f)\,df$$

La puissance du signal peut alors être extraite de l'analyse fréquentielle telle qu'illustrée en figure 10.

L'analyse de variabilité du rythme cardiaque peut également être effectuée sur d'autres paramètres, notamment des données temporelles (RMSSD, SDNN, SDANN, etc.), des valeurs géométriques telles que la distribution de densité NN, la courbe de Lorenz et des mesures non linéaires telles que les approches fractales ou la courbe dite de Poincaré. L'ensemble de ces analyses peut être implanté dans un dispositif médical implantable et mis en oeuvre selon l'invention décrite.

**Revendications**

**1.** Dispositif médical implantable actif (1) comprenant une unité de traitement (2) apte à être mise alternativement en fonctionnement durant une période d'activité déterminée et en veille durant une période de veille de manière cyclique, et des moyens d'acquisition de données (16) liées à l'activité physiologique et/ou physique,
**caractérisé en ce que** le dispositif comprend en outre
des moyens de calcul d'une analyse fréquentielle (17) des données acquises, lesdits moyens de calcul étant aptes à exécuter successivement une partie de l'analyse fréquentielle durant des périodes d'activité de l'unité de traitement, et
des moyens d'optimisation de l'analyse fréquentielle (18) par fractionnement de l'analyse fréquentielle en une pluralité de modules élémentaires, les moyens de calcul (17) exécutant successivement les modules élémentaires durant des périodes d'activité de l'unité de traitement.

**2.** Dispositif selon la revendication 1 , **caractérisé en ce que** les moyens de calcul sont mis en oeuvre au cours de la période d'activité de l'unité de traitement après exécution des fonctions standards de traitement exécutées durant la période d'activité de l'unité de traitement.

3. Dispositif selon la revendication 2, **caractérisé en ce que** les moyens de calcul (17) exécutent une partie de l'analyse fréquentielle après exécution des fonctions standards de traitement exécutées jusqu'à la fin de la période d'activité de l'unité de traitement.

4. Dispositif selon la revendication 1 ou la revendication 2, **caractérisé en ce que** lesdits moyens de calcul (17) exécutent une partie de l'analyse fréquentielle durant une durée prédéterminée.

5. Dispositif selon la revendication précédente, **caractérisé en ce que** les moyens d'optimisation de l'analyse fréquentielle (18) sont aptes en outre à sélectionner au moins une bande de fréquence à traiter.

6. Dispositif selon l'une des revendications précédentes, **caractérisé en ce que** le dispositif comprend en outre des moyens de sélection d'une période d'acquisition (19) desdites données.

7. Dispositif selon la revendication 6, **caractérisé en ce que** les moyens de sélection d'une période d'acquisition (19) comprennent en outre des moyens de vérification de conformité de critères prédéterminés.

8. Dispositif selon la revendication 6 ou la revendication 7, **caractérisé en ce que** les moyens d'acquisition des données (16) sont mis en oeuvre lorsque la période d'acquisition est atteinte et que les critères sont conformes à des valeurs prédéterminées.

9. Dispositif selon l'une des revendications précédentes, **caractérisé en ce que** le dispositif comprend en outre des moyens de détection d'un événement physiologique déterminé externe au dispositif et des moyens de démarrage du cycle apte à démarrer le cycle lorsque les moyens de détection détectent un événement physiologique déterminé.

10. Dispositif selon l'une des revendications précédentes, **caractérisé en ce que** le dispositif comprend en outre des moyens de prétraitement (20) des données acquises.

11. Dispositif selon la revendication 10, **caractérisé en ce que** les moyens de prétraitement (20) sont aptes à interpoler et rééchantillonner les données acquises.

12. Dispositif selon l'une quelconque des revendications précédentes, **caractérisé en ce que** les moyens de calcul (17) sont mis en oeuvre dans une partie finale de la période d'activité de l'unité de traitement.

13. Dispositif selon l'une quelconque des revendications précédentes, **caractérisé en ce que** l'analyse fréquentielle est une transformation de Fourier.

**Patentansprüche**

1. Aktive implantierbare medizinische Vorrichtung (1) umfassend eine Verarbeitungseinheit (2), die zyklisch abwechselnd während eines vorbestimmten Betriebszeitraums in Betrieb genommen und während eines Ruhezeitraums in einen Ruhezustand versetzt werden kann, sowie Mittel (16) zur Erfassung von mit der physiologischen und/oder physischen Aktivität verbundenen Daten,
   **dadurch gekennzeichnet, dass** die Vorrichtung weiter Folgendes umfasst:

   Mittel (17) zur Berechnung einer Frequenzanalyse der erfassten Daten, wobei diese Berechnungsmittel fähig sind, einen Teil der Frequenzanalyse während Betriebszeiträumen der Verarbeitungseinheit nacheinander durchzuführen, und
   Mittel (18) zur Optimierung der Frequenzanalyse durch eine Aufteilung der Frequenzanalyse in eine Vielzahl von Elementarmodulen, wobei die Berechnungsmittel (17) während Betriebszeiträumen der Verarbeitungseinheit die Elementarmodule nacheinander ausführen.

2. Vorrichtung nach Anspruch 1, **dadurch gekennzeichnet, dass** die Berechnungsmittel während des Betriebszeitraums der Verarbeitungseinheit, nach Durchführung der Standard-Verarbeitungsfunktionen während des Betriebszeitraums der Verarbeitungseinheit, eingesetzt werden.

3. Vorrichtung nach Anspruch 2, **dadurch gekennzeichnet, dass** die Berechnungsmittel (17) einen Teil der Frequenzanalyse nach Durchführung der Standard-Verarbeitungsfunktionen durchführt, die bis zum Ende des Betriebszeitraums der Verarbeitungseinheit durchgeführt werden.

4. Vorrichtung nach Anspruch 1 oder Anspruch 2, **dadurch gekennzeichnet, dass** die Berechnungsmittel (17) einen Teil der Frequenzanalyse während eines vorbestimmten Zeitraums durchführen.

5. Vorrichtung nach dem vorhergehenden Anspruch, **dadurch gekennzeichnet, dass** die Mittel (18) zur Optimierung der Frequenzanalyse fähig sind mindestens ein zu verarbeitendes Frequenzband auszuwählen.

6. Vorrichtung nach einem der vorhergehenden Ansprüche, **dadurch gekennzeichnet, dass** die Vorrichtung außerdem Mittel (19) zur Auswahl eines Zeitraums zur Erfassung der Daten umfasst.

7. Vorrichtung nach Anspruch 6, **dadurch gekennzeichnet, dass** die Mittel (19) zur Auswahl eines Erfassungszeitraums außerdem Mittel zur Überprüfung der Konformität von vorbestimmten Kriterien umfassen.

8. Vorrichtung nach Anspruch 6 oder Anspruch 7, **dadurch gekennzeichnet, dass** die Mittel (16) zur Datenerfassung eingesetzt werden, wenn der Erfassungszeitraum erreicht wird und die Kriterien mit vorbestimmten Werten übereinstimmen.

9. Vorrichtung nach einem der vorhergehenden Ansprüche, **dadurch gekennzeichnet, dass** die Vorrichtung außerdem Mittel zur Erfassung eines bestimmten, außerhalb der Vorrichtung stattfindenden physiologischen Ereignisses umfasst, sowie Mittel zum Starten des Zyklus, die fähig sind den Zyklus zu starten, wenn die Erfassungsmittel ein bestimmtes physiologisches Ereignis erfassen.

10. Vorrichtung nach einem der vorhergehenden Ansprüche, **dadurch gekennzeichnet, dass** die Vorrichtung außerdem Mittel zur Vorverarbeitung (20) der erfassten Daten umfasst.

11. Vorrichtung nach Anspruch 10, **dadurch gekennzeichnet, dass** die Vorverarbeitungsmittel (20) fähig sind die erfassten Daten zu interpolieren und erneut abzutasten.

12. Vorrichtung nach einem der vorhergehenden Ansprüche, **dadurch gekennzeichnet, dass** die Berechnungsmittel (17) in einem letzten Teil des Betriebszeitraums der Verarbeitungseinheit eingesetzt werden.

13. Vorrichtung nach einem der vorhergehenden Ansprüche, **dadurch gekennzeichnet, dass** die Frequenzanalyse eine Fourier-Transformation ist.

**Claims**

1. Active implantable medical device (1) comprising a processing unit (2) able to be alternately operated during a determined period of activity and on standby during a period of standby in a cyclical manner, and means (16) for acquiring data related to a physiological and/or physical activity,
**characterized in that** the device further comprises calculating means (17) for calculating a frequency analysis of the acquired data, said calculating means being able to successively execute a portion of the frequency analysis during periods of activity of the processing unit, and
means (18) for optimizing the frequency analysis by fractioning the frequency analysis into a plurality of elementary modules, the calculating means (17) successively executing the elementary modules during periods of activity of the processing unit.

2. Device according to claim 1, **characterized in that** the calculating means are implemented during the period of activity of the processing unit after execution of the standard processing functions executed during the period of activity of the processing unit.

3. Device according to claim 2, **characterized in that** the calculating means (17) execute a portion of the frequency analysis after execution of the standard processing functions executed until the end of the period of activity of the processing unit.

4. Device according to claim 1 or claim 2, **characterized in that** said calculating means (17) execute a portion of the frequency analysis for a predetermined duration.

5. Device according to the preceding claim, **characterized in that** the means (18) for optimizing the frequency analysis are also able to select at least one frequency band to be processed.

6. Device according to one of the preceding claims, **characterized in that** the device further comprises means (19) for selecting a period of acquisition of said data.

7. Device according to claim 6, **characterized in that** the means (19) for selecting an acquisition period further comprises means for monitoring the conformity of predetermined criteria.

8. Device according to claim 6 or claim 7, **characterized in that** the means (16) for acquiring data are implemented when the period of acquisition is reached and the criteria are in accordance with predetermined values.

9. Device according to one of the preceding claims, **characterized in that** the device further comprises means for detecting a determined physiological event external to the device and means for starting the cycle able to start the cycle when the detection means detect a specific physiological event.

10. Device according to one of the preceding claims, **characterized in that** the device further comprises preprocessing means (20) of the acquired data.

11. Device according to claim 10, **characterized in that** the preprocessing means (20) are capable of interpolating and resampling the acquired data.

12. Device according to one of the preceding claims, **characterized in that** the calculating means (17) are implemented in a final portion of the period of activity of the processing unit.

13. Device according to one of the preceding claims, **characterized in that** the frequency analysis is a Fourier transform.

# Fig.1

8a

9

8b

12

3

10

11

2

4

5

1

6

13a

13b

13c

13d

13

7

15

14

17

16

19

18

20

# Fig.2

21 Sélection d'une période

22 Acquisition de données

23 Prétraitement

24 Optimisation de l'analyse

25 Calcul d'une analyse

EP 3 150 252 B1

# Fig.3

# Fig.4

# Fig.5

me me me me me

# Fig.6

pr pr pr pr pr

# Fig.7

pc pc pc pc pc

# Fig.8

71

81

82  non

oui

83  non

oui

84

non  85

oui

72

73

86  non

oui

87

88

89

74

90

91

92

93  oui

non

94

## Fig.9

## Fig.10

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20150257669 A1
- US 20070260285 A1
- US 20140088379 A1
- US 20070255331 A1
- WO 9119452 A
- US 7186220 B
- FR 2492262